# EUROPEAN PATENT APPLICATION

(11) **EP 2 520 334 A1**
(43) Date of publication of application: **07.11.2012**
(21) Application number: 11003580.5
(22) Date of filing: 02.05.2011
(51) Int. Cl.: A61N 2/00, A61N 2/02, A61N 1/40, A61N 1/378, A61N 1/39

(54) **Magnetic stimulation using plasma**

(71) Applicant: Magnetic Pacing Technologies GmbH, 47057 Duisburg (DE)
(72) Inventor: Kisker, Erhard, 40627 Düsseldorf (DE)
(74) Representative: Häckel, Stefan

(57) **Abstract**

The present invention provides a stimulation device adapted to directly or indirectly stimulating a tissue by means of a time-varying magnetic field, wherein the stimulation device is adapted to generate a time-varying magnetic field by means of a current flow through a plasma.

## Description

The present invention relates to a stimulation device for stimulating a tissue, to a stimulation system comprising a stimulation device and to the use of a current conducting plasma and/or superconductor.

The present invention generally relates to directly or indirectly stimulating a tissue or organ by means of a time-varying magnetic field. However, the invention can be applied to stimulation of different materials such as samples in nuclear magnetic resonance imaging or to electrochemical analysis as well.

Direct stimulation can be achieved by applying the magnetic field to a conducting material, in particular a tissue, causing a current and corresponding electric fields for stimulation. Kubuta et al., "High-Performance Stimulating Coils and Eddy Currents in Magnetic Heart Stimulation", Japanese Journal of Applied Physics, Volume 34 (1995), pp. 5877 - 5883, describes direct magnetic stimulation by means of a pulsed magnetic field.

US 2009 024 180 A1 relates to a stimulation system comprising an implanted electrode device with electrodes for stimulating a heart using an electrical impulse. A magnetic field is generated by a control device acting on the electrode device for stimulating a tissue by means of an electrical impulse provided by the electrode device.

Magnetic fields can be provided by coils placed close to or inside a body of a patient whose tissue is to be stimulated. However, common coils make use of copper wires which are expensive and heavy. Furthermore, a maximum magnetic field strength depends on the coil volume and weight. Thus, mobile (transportable) stimulation devices and, in particular, implantable stimulation devices suffer from a maximum field strength that is limited by the weight and volume of the coil.

Object of the present invention is to provide a stimulation device, a stimulation system and a use which allow for stimulation by means of a time-varying magnetic field, wherein the stimulation device can be realized smaller, cheaper and/or lighter.

This object is achieved by a stimulation device according claim 1, by a stimulation system according to claim 14 or by a use according to claim 15. Advantageous embodiments are subject of the subclaims.

A stimulation device according to the present invention is adapted to directly or indirectly stimulating a tissue by means of a time-varying magnetic field, wherein the stimulation device is adapted to generate the time-varying magnetic field by means of a current flow through a plasma and/or superconductor.

In the sense of the present invention, plasma is a material comprising particles which are ionized. These ions are acting as charge carriers making the plasma electrically conductive. A current flow through a conductor causes a magnetic field. Thus, a current flow through the plasma can be used for generating magnetic fields. According to the present invention, such a current flow through plasma is used to generate a time-varying magnetic field suitable for stimulating a tissue. Plasma has a low resistance and its mass is vanishing compared to metals like copper at the same current carrying capacity. Thus, using plasma for generating the time-varying magnetic field allows for a lighter stimulation device. Furthermore, less copper is needed and, thus, the time-varying magnetic field can be achieved with a less expensive stimulation device.

Plasma can be generated from a gas or gaseous material, in particular by current flow between two electrodes, due to and/or after ignition. This is a strongly non-linear process. The conductance switches from a very low value to a very large value on a µs time scale, in particular from several MΩ to less than 10 Ω, pereferably 5 Ω and/or in less than 10 µs, preferably less than 5 µs. Thereby the current increases from virtually zero to a very high value determined by the voltage between the electrodes. The increase in current is to one part limited by the self-inductance of the plasma. The latter is determined by geometry. The geometry can be kept simple by using only one or a few circular windings or a linear shape providing a very small inductance allowing for of high di/dt rates, i.e. high values of a time derivative of the current, and corresponding high dH/dt rates, i.e. high values of a time derivation or high amplitudes of the time-varying magnetic field strength. Considering Faraday's law, the variation rate dH/dt of a magnetic field is proportional to the variation rate di/dt of the current causing the magnetic field. A high di/dt and/or dH/dt rate advantageously supports stimulation by means of the time-varying magnetic field. Thus, the stimulation efficiency can benefit from using plasma.

According to one aspect of the present invention, generating the plasma can be used for controlling the variation of the time-varying magnetic field, in particular by using the variation due to the starting process and/or by varying the starting conditions. Due to nonlinear effects including negative resistance behavior of the plasma, in particular at start of conduction and/or after initial generation of the plasma, the current through the plasma can be increased very fast leading to high di/dt rates. This further can be supported by the low inductance of the plasma, in particular the plasma pinch. This enables controlling the variation of the time-varying magnetic field and achieving very high di/dt rates simultaneously. High di/dt rates and/or corresponding dH/dt rates are necessary for improving the stimulation efficiency.

In particular, using plasma allows for cost reduction, because in known solutions defibrillation attempts for achieving large di/dt rates, fast and expensive electronic switches are required in order to achieve large di/dt rates. Furthermore, the plasma can be used as a switch, in particular by means of controlling generation of the plasma by which energy losses in a separate switch can be omitted.

It is particularly preferred that plasma and/or superconductors are used by the stimulation device for generating the time-varying magnetic field. In particular, use of plasma and/or superconductors allow for producing more powerful magnetic field gradients, i.e. a faster variation of magnetic field strength, compared to conventional conductors, e.g. solid metals.

In particular, using high-temperature superconductors materials is particularly preferred. This allows for less cooling effort and optionally for implanting. Today's high-temperature superconductor materials (Cuprates) require cooling down to ∼100K or less. For stationary defibrillators with access to a main power grid continuous cooling can be provided. Thus, using superconductors is particularly preferred for stationary stimulation devices, in particular defibrillators, preferably adapted for direct stimulation. However, small and/or transportable stimulation devices using superconductors are in focus of the present invention as well, wherein future superconductors working at or close to room temperature are appreciated.

Superconductors in the sense of the present invention have an electrical resistance of about zero below a characteristic temperature. Superconductivity is a quantum mechanical phenomenon. Typically, electrical resistivity of metallic conductors decrease by reducing temperature, which is limited by impurities. Even at about absolute zero, metals like copper show some resistance. In contrast, the resistance of a superconductor drops abruptly to about zero if the material is cooled below its critical temperature. An electric current flowing in a loop of superconducting wire can, thus, persist almost indefinitely with no power source. High temperature superconductors in the sense of the present invention provide a critical temperature higher than the boiling point of liquid nitrogen (77 K or -196 °C).

According to a first aspect of the present invention, the stimulation device is adapted for controlling and/or supplying with energy of an implanted electrode device (satellite) in an exclusively wireless manner by means of this time-varying magnetic field. In particular, the stimulation device is arranged and designed in such a way that it can control and/or supply an implanted electrode device, i.e., the stimulation device can generate time-varying magnetic fields of characteristics which allow for affecting the implanted electrode device. For instance, control commands and/or energy can be provided by the stimulation device for controlling and/or supplying the electrode device, respectively.

According to a second aspect of the present invention, that can be realized independently as well, the stimulation device is a defibrillator adapted for depolarization of cells by means of the time-varying magnetic field generated with the current flowing through the plasma and/or superconductor. In particular, this magnetic field is used for direct stimulation, e.g. by directly generating an electrical field in the conducting material (tissue) with this magnetic field. This allows for a lightweight and transportable defibrillator and/or a defibrillator that does not need applying electrodes to the body.

The stimulation device according to the present invention can be used either stand alone, in particular as a defibrillator or as a cardiac pacemaker, or in combination with or forming an inductively sourced pacemaker, in particular with an electrode device. In particular, the stimulation device according to the present invention provides both a defibrillator and pacemaker function. For instance, in the pacemaker mode the operating conditions for the plasma or the vessel can be chosen or controlled such that generating the time-varying magnetic field provides an induction voltage sufficient for stimulation by means of the implanted electrode device. Alternatively or additionally, the stimulation device is adapted for detecting need of defibrillation.

The stimulation device can provide a defibrillation mode, preferably which can be activated on demand. In the defibrillation mode, the control device in particular controls and/or modifies the operating conditions for the plasma or the vessel, in particular by means of a electric field acting on the plasma, preferably such that the resistance of the plasma is reduced and/or such that a variation burst of the time-vaying magnetic field is generated, in particular with a high and/or alternating di/dt value. This can allow for stimulating the heart, tissue or the like directly by means of an electric field generated due to Faraday's law. Another aspect of the present invention is a mobile or transportable defibrillator working because of Faraday's law without the need of placing electrodes.

A further aspect, that can be realized independently as well, relates to a stimulation device adapted to directly or indirectly stimulating a tissue by means of a time-varying magnetic field, wherein the stimulation device is adapted to generate the time-varying magnetic field by means of a current flow through the plasma inside a plasma vessel, wherein the vessel is adapted to block or reduce emission of electromagnetic radiation, in particular by means of a shielding layer and/or coating. A plasma typically emits electromagnetic radiation, in particular light, UV or X-rays. However, the present invention focuses on generating the time-varying magnetic field by the current flowing through the plasma. Electromagnetic radiation, in particular light emission, therefore might not be intended since it might cause irritations. Thus, reducing electromagnetic radiation by means of shielding according to the present invention allows for focusing on generating magnetic fields and can increase the efficiency of the magnetic field generation since radiation losses can be reduced.

Additionally or alternatively, ionizable materials are used for generating the plasma, wherein these materials allow for generation of a plasma causing less emissions compared to common inert gas. Alternatively or additionally, the plasma can be controlled, in particular pulsed, such that the plasma emits less electromagnetic radiation.

According to a further aspect of the present invention, the vessel can comprise glass or different, preferably insulating, material, which preferably is lightweight and leads to a lightweight stimulation device as well.

A further aspect of the present invention, that can be realized independently as well, relates to a stimulation system comprising at least one implantable electrode device and a stimulation device adapted to directly or indirectly stimulating a tissue by means of a time-varying magnetic field, wherein the stimulation device is adapted to generate the time-varying magnetic field by means of a current flow through a plasma. The implantable electrode device comprises electrodes for delivery of an electrical impulse to a surrounding tissue, wherein the electrode device is adapted to be controlled and/or supplied with energy exclusively in a wireless manner by means of the time-varying magnetic field of the stimulation device. The stimulation system according to the present invention in particular benefits from the cheap and lightweight stimulation device which makes use of the current flowing through the plasma for generating the magnetic field. Thus, the stimulation system can be realized cheap and lightweight as well.

A further aspect of the present invention, that can be realized independently as well, relates to the use of a current conducting plasma for generating a time-varying magnetic field and for controlling and/or supplying with energy of a remote device, in particular an electrode device as described above, in an exclusively wireless manner by means of this time-varying magnetic field.

A remote device can be realized as a device in production or analysis technology. For instance, a remote device can be an electrode device, in particular for electroanalysis of a reagent in a closed environment. Such an electrode device can be controlled and/or supplied in an exclusively wireless manner by means of the time-varying magnetic field which is generated by means of the current flowing through the plasma. Thus, the electrode device according to the present invention is not limited to the purpose of stimulation but can be applied to different fields of technology as well.

The above and following aspects of the present invention can be realized separately and in any combination. In particular, a direct stimulation and a stimulation by means of an electrode device can be combined and/or realized in a shared stimulation system.

Further advantages, properties, features and aspects of the present invention will become apparent upon review of the claims and the following description of preferred embodiments with reference to the drawings.

In the figures:
- Fig. 1: is a schematic view of an inventive stimulation system;
- Fig. 2: is a cross-sectional view of a plasma vessel according to one embodiment of the present invention;
- Fig. 3: is a cross-sectional view of a plasma vessel according to a further embodiment of the present invention;
- Fig. 4: is a simplified schematic view of an inventive stimulation system placed inside the body of a patient; and
- Fig. 5: is a simplified schematic view of a stationary stimulation system according to the present invention.

In the figures, the same reference numerals are used for the same parts or parts of the same type, components or the like, wherein corresponding or similar advantages and properties can be obtained even if the description is not repeated. Further, the invention is discussed primarily referring to plasma. However, preferably it is to be understood that aspects discussed in the following can apply to superconductors instead or in addition to plasma as well.

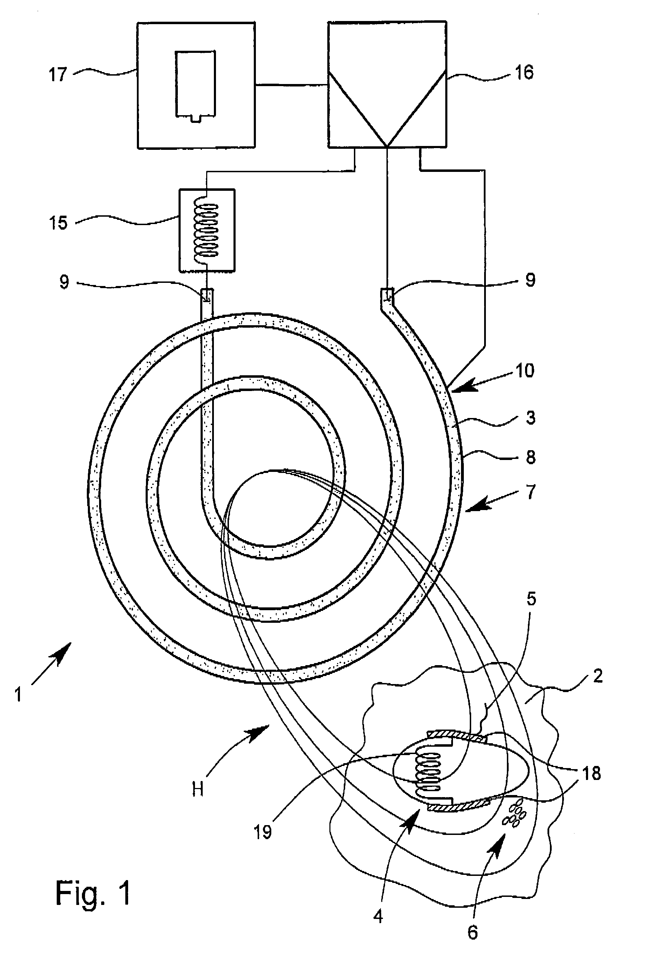

Fig. 1 shows a stimulation device 1 adapted to directly or indirectly stimulating a tissue 2 by means of a time-varying magnetic field H. The stimulation device 1 is adapted to generate the time-varying magnetic field H by means of a current I flowing through a plasma 3, and/or a superconductor.

In the sense of the present invention, the term "time-varying magnetic field H" preferably covers any field or (electromagnetic) wave comprising at least a magnetic or magnetic field component.

The stimulation device 1 can be adapted for indirect stimulation by sourcing and/or controlling a remote device, in particular an electrode device 4, in the following preferably referred to as indirect stimulation, which is discussed in the following. Alternatively or additionally, the stimulation device 1 can be adapted for direct stimulation by inducing a current, eddy current, voltage, or an electric field directly to the material or tissue 2 to be stimulated, in the following preferably referred to as direct stimulation, which will be discussed later.

The stimulation device 1 can be adapted for supplying an implantable electrode device 4 with energy and/or for controlling an implantable electrode device 4, e.g., by means of characteristics of the time-varying magnetic field H, in particular by a coded time-varying magnetic field H. The electrode device 4 preferably is implantable, in particular by means of a watertight, biocompatible casing and/or a holding means for fixing its position.

The stimulation system in the example shown in Fig. 1 comprises a stimulation device 1 adapted to generate the time-varying magnetic field H by means of the current I flowing through the plasma 3 and additionally comprises at least one implantable electrode device 4 with electrodes 18 for delivering electrical impulses 5, in particular to the surrounding tissue 2. The electrode device 4 preferably is adapted to be controlled and/or supplied with energy exclusively in a wireless manner by means of the time-varying magnetic field H of the stimulation device 1.

In particular, the electrode device 4 can comprise a receiving coil 19 for receiving energy provided by the time-varying magnetic field H and to generate the electrical impulse 5.

Optionally, electrode device 4 can comprise an impulse forming means for controlling the shape and/or duration of the electrical impulse 5 and/or energy storing means that can be charged by means of the time-varying magnetic field H prior to generation of an electrical impulse 5.

Furthermore, electrode device 4 can comprise means for triggering delivery of the electrical impulse 5, preferably by means of the time-varying magnetic field H. The control device 1 can comprise means for selectively supplying with energy and/or controlling different electrode devices 4, e.g. by means of different transmission characteristics of the time-varying magnetic field H, in particular different frequencies, polarities or the like.

Using the electrode device 4, the stimulation device 1 can stimulate indirectly by sourcing and/or controlling the separate electrode device 4 placed remote form the stimulation device 1 in a wireless manner by means of the time-varying magnetic field H. This enables the electrode device 4 for stimulation, e.g., by delivering an electrical impulse 5 via electrodes 18. More than one electrode device 4 can be provided, preferably wherein this discussion applies to each electrode device 4.

The time-varying magnetic field H is generated by the stimulation device 1 preferably such that the time-varying magnetic field H reaches and affects the electrode device 4. The stimulation device 1 and the electrode device 4 preferably are placed within a distance of less than 15 cm, preferably less than 10 cm, in particular less than 5 cm. The time-varying magnetic field H can induce a current in the electrode device 4, in particular in a receiving coil 19, wherein the current preferably can be used by the electrode device 4 for generating or can act as an electrical impulse 5 for stimulation.

The stimulation device 1 preferably is a defibrillator or a part of it. In the sense of the present invention, the term "defibrillator" preferably relates to a device which is adapted for defibrillation by means of depolarization of cells 6. According to the present invention, defibrillation is performed with the time-varying magnetic field H, preferably either directly or indirectly via electrode device 4 or the like. In particular, a defibrillation in the sense of the present invention is a depolarization of more than 70 % of the tissue of a heart.

The time-varying magnetic field H can be used to supply the preferably implantable electrode device 4 with energy and the electrode device 4 provides an electrical impulse 5. This electrical impulse 5 can be used for stimulation and, in particular, for defibrillation. However, this electrical impulse 5 can be used for different purposes, in particular for electroanalysis, electrolysis or the like, as well.

According to one aspect of the present invention, stimulation device 1 is used for direct stimulation. Stimulation device 1 preferably is adapted to generate the time-varying magnetic field H comprising at least one magnetic field pulse for direct stimulation, in particular defibrillation. This pulse can influence, preferably stimulate, in particular depolarize, the tissue 2, in particular of a cardiac muscle (cardioversion) and/or by producing eddy currents in the vicinity of a heart or other tissues 2 like nerves of a brain or the like. Thus, the stimulation device 1 can act as a defibrillator by direct stimulation, which in the following is referred to as "magnetic defibrillation".

If direct stimulation, in particular magnetic defibrillation, is desired, the stimulation device 1 preferably is adapted to generate a time-varying magnetic field H of a sufficiently high variation in field strength. Preferably, at least one pulse is generated capable of generating an electrical field of a field strength that directly leads to a depolarization of cells 6 of the tissue 2, in particular of a heart.

Time-varying magnetic field H in tissue 2 leads to an electric field, in particular according to the Maxwell equations. The field strength of this electric field for depolarization and/or magnetic defibrillation preferably exceeds 20 V/m, in particular 30 V/m. It is preferred that the stimulation device 1 for magnetic defibrillation provides a time-varying magnetic field H with a peak flux of more than 5 T, preferably more than 8 T, in particular more than 10 T. The flux variation provided by the time-varying magnetic field H preferably exceeds 3 kT/s, in particular 5 kT/s or 7 kT/s. Additionally or alternatively, it is preferred that the time-varying magnetic field H comprises a pulse with a duration preferably of more than 2 ms, in particular 2,5 ms. This allows for reaching the intended minimum electric field strength and/or a reliable defibrillation.

The stimulation device 1 can be adapted to sense an electrical behavior and for triggering generation of the time-varying magnetic field H synchronized with the sensing result. In particular, the time-varying magnetic field H can be synchronized with an electrocardiogram signal of a heart sensed by the stimulation device 1, e.g. via electrodes of the stimulation device 1, in particular if pacing of the heart is desired. Alternatively or additionally, a time-varying magnetic field pulse for magnetic pacing can be generated by the stimulation device 1 if an arrhythmia or the like is detected. However, stimulation device 1 additionally or alternatively can sense a temperature, an electrical behavior or the like for synchronization in different technical fields as well, e.g. for control of an electrolysis or electroanalysis of a sample.

The stimulation device 1 can provide a pacing mode and a defibrillation mode simultaneously, intermittingly or alternatively. The stimulation device 1 can be adapted to distinguish between or can provide a pacing mode with a reduced amplitude of the field strength or variation amplitude of time-varying magnetic field H and a magnetic defibrillation mode with a higher magnetic field strength or variation amplitude. In particular, in the pacing mode a peak flux provided be the time-varying magnetic field is less than 5 T, in particular 2 T and/or a maximum flux variation is less than 3 kT/s, in particular less than 1 kT/s. The flux variation provided by the time-varying magnetic field H in the defibrillation mode preferably exceeds 3 kT/s, in particular 5 kT/s or 7 kT/s.

Alternatively or additionally, the time-varying magnetic field H can transfer energy over a longer time span in defibrillation mode than in the pacing mode. In a pacing mode, stimulation can be provided indirectly by means of electrode device 4, wherein defibrillation is provided in a direct stimulation manner, or vice versa. Thus, stimulation device 1 can be adapted to supply a remote device, e.g. the electrode device 4, in particular for pacing, electrolysis or electroanalysis, and to direct stimulation, in particular defibrillation or for heating a sample by eddy currents, simultaneously, intermittingly or alternatively.

In a preferred embodiment, stimulation device 1 is implantable. Implanting the stimulation device 1 enables placing closer to the area the time-varying magnetic field H is to be applied to and the efficiency, thus, can be increased. Implanting stimulation device 1 preferably is preferred if the electrode device 4 is to be supplied with energy and/or controlled.

According to the present invention, a general idea is to use plasma 3 and/or a superconductor that conducts current I and thereby generates the time-varying magnetic filed H. Using plasma 3 is well known in the fields of emission of light using fluorescent lamps or for plasma etching. In contrast, the present invention makes use of plasma 3 due to its electrical conductivity.

One aspect of the present invention, which can be realized separately as well, relates to using plasma 3 for generation of a time-varying magnetic field H, wherein the plasma 3 is shielded such that emission of electromagnetic radiation is reduced. This allows for efficiency enhancement since energy losses due to electromagnetic radiation is omitted. In the following, this aspect will be discussed referring to preferred embodiments of stimulation device 1 preferably comprising shielding means capable of reducing emission of electromagnetic radiation.

Ionized particles forming plasma 3 can be hot or reactive and, thus, preferably are separated from the surrounding area. It is preferred that the stimulation device 1 comprises a plasma vessel 7 for separation of the plasma 3, in particular wherein the vessel 3 encloses and/or seals the plasma 3. Vessel 7 can be filled with an ionizable gaseous medium, in particular an inert gas and/or a metal vapor. This ionizable gaseous medium can form the plasma when or if it is at least partially ionized.

The vessel 7 surrounding the plasma can either be similar to a circular loop comprising more than 1 (n) or a straight tube of length more than 1 cm, preferable more than 10 cm long. For achieving optimal spatial distribution of the induced field, several of these vessels can be combined to act together. The individual plasma might be generated separately for achieving a gradient of the electric field. Such a gradient can improve the stimulation efficiency.

The vessel 7 preferably comprises an electrical insulating wall 8 forming a gas tight interior of the vessel 7. The vessel 7 further can comprise at least two connecting electrodes 9 at the lengthwise opposing ends of the vessel 7. These connecting electrodes 9 electrically connect the plasma 3 and/or the interior to the exterior of the vessel 7. Optionally, the vessel 7 can comprise an additional ionization electrode 10 for ionization of the ionizable gaseous medium and/or for generating the plasma 3. The ionization electrode 10 preferably is arranged outside the vessel 7 and/or electrically insulated from the interior of the vessel 7 and is adapted to ionize the gaseous medium inside the vessel 7, in particular by means of an electric field. For example, the ionization electrode 10 can be formed by a conductor placed close to the outer surface of the vessel 7, in particular by a lead wound around the vessel 7. In a preferred alternative, ionization electrode 10 is formed by the shielding means of stimulation device 1.

The vessel 7 and/or the plasma 3 preferably is or are wound, in particular comprising at least two or three turns. In particular, the turns of the vessel 7 have diameter of more than 2 cm, preferably more than 3 cm, in particular more than 5 cm, and/or less than 30 cm, preferably less than 20 cm, in particular less than 15 cm. This allows for a compact and efficient stimulation device 1. A helix-shaped vessel 7 is depicted in Fig. 1. However, this helix-shape of the vessel 7, plasma 3 and/or superconductor can be replaced by different topologies known in the art, in particular by cylindrical, linear and/or multilayer coil structures formed by vessel 7. Particularly preferably the shape can be at least substantially linear. This provides minimum self-inductance values which support high variation rates of the current I and/or time-varying magnetic field H. Further, the shape can be adapted and/or multiple, preferably at least substantially linear, vessels 7 can be used such that an electrical field gradient can be achieved in the body B or sample to be stimulated. This can improve the stimulation efficiency.

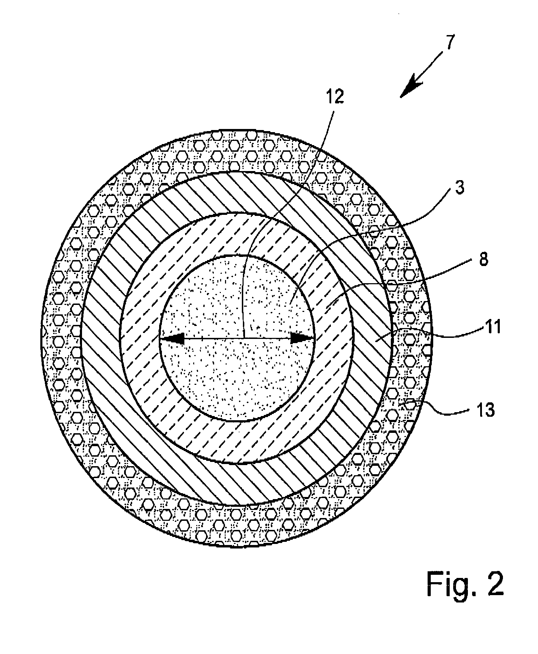

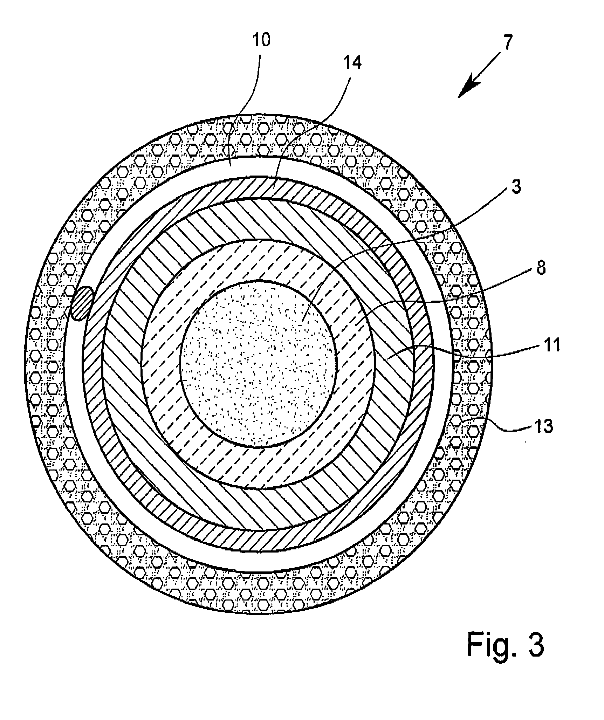

Vessel 7, plasma 3 and/or the superconductor can comprise different diameters. However, some or all turns can have the same diameter and/or can form a different coil alternatively or additionally. Vessel 7 can be flexible and/or break proof, which advantageously supports implantability of the vessel 7 and the stimulation device 1. Vessel 7 preferably is elongate, in particular wherein its length factor of more than 10, preferably 20, higher than its diameter and/or the vessel 7 is wound and the windings formed by vessel 7 have an diameter a factor of more than 10, preferably 20, higher than the diameter, in particular an inner diameter 12, of the vessel 7 in the cross-section (Fig. 2 and 3).

In the following, vessel 7 is described in further detail using Fig. 2 and 3 showing cross-sectional views of vessels 7 according to preferred embodiments.

Vessel 7 in Fig. 2 comprises electrically insulating wall 8 that preferably is gas tight and encloses plasma 3, such that plasma 3 cannot leave the vessel 7. Vessel 7 preferably is adapted to block or reduce emission of electromagnetic radiation. In particular, a layer 11 of the vessel 7 can act as a shielding means capable of reducing emission of electromagnetic radiation. The shielding means and/or layer 11 preferably blocks, converts, shields and/or reflects light, UV, X-rays, Gamma rays and/or radiations with frequencies higher than 10⁹ Hz, preferably higher than 10¹¹ Hz, in particular higher than 10¹³ Hz and/or lower than 10¹⁸ Hz, preferably lower than 10¹⁷ Hz, in particular lower than 10¹⁶ Hz. Thus, emission of energy different than emission by the time-varying magnetic field H is reduced or avoided. Layer 11 preferably covers the interior of the vessel 7 at least substantially. This allows for an improved shielding efficiency.

In Fig. 2, layer 11 preferably is electrically conducting and forms the ionization electrode 10. Layer 11 preferably comprises a material that conducts electrically and/or blocks, converts, shields and/or reflects electromagnetic radiation. Thus, layer 11 shields electromagnetic radiation and acts as an ionization electrode 10, simultaneously.

In the example shown, layer 11 is an exterior coating of the vessel 7 and/or is placed outside the wall 8, i.e., at the side of the wall 8 which is remote from the plasma 3. Layer 11 can comprise metal and/or can be applied to the wall 8 by sputtering, vapor deposition or the like.

Vessel 7 in the cross-section shown in Fig. 2 or 3 has an inside diameter 12 of less than 5 mm, preferably less than 2 mm, in particular less than 1 mm. This inside diameter 12 preferably corresponds to a hydraulic diameter. Further, the inside diameter 12 preferably relates to diameter formed by an inner surface of wall 8 in the cross-sectional view of vessel 7.

Vessel 7 preferably comprises a cover 13 at the side of layer 11 and/or wall 8 far from plasma 3. In particular, plasma 3 is surrounded by wall 8 which is surrounded by layer 11 and, preferably, wall 8 and/or layer 11 is or are surrounded by cover 13. Cover 13 preferably is tissue-friendly and biocompatible. Thus, implanting the stimulation device 1 is supported. Optionally, vessel 7 or stimulation device 1 can comprise a holding means for anchoring in the implanted state.

Fig. 3 shows a cross-section of vessel 7 according to a different embodiment, wherein ionization electrode 10 and layer 11 are realized separately. In the example shown, ionization electrode 10 is wound around layer 11, in particular formed by a lead. The ionization electrode 10 can be attached to layer 11 or can be separated therefrom, e.g., by an insulator 14. This in particular allows for using a layer 11 that is insulating or has a high resistivity.

Further optional components of stimulation device 1 are shown in Fig. 1. The stimulation device 1 can comprise a coil 15, wherein the plasma 3 and/or the interior of vessel 7 is electrically connected via this coil 15. In particular, coil 15 is realized as a choke. In the sense of the present invention, a choke is a coil suitable for limiting a maximum current or variation of a current I through the plasma 3.

The plasma 3 preferably has a low resistivity of less than 10 Ω, preferably less than 5 Ω, in particular less than 2 Ω. If energy available for generating the time-varying magnetic field H is limited, a maximum duration the time-varying magnetic field H can be generated is limited as well. The power consumption depends on the voltage provided to plasma 3 as well as on the resistivity of plasma 3. Coil 15 allows for reducing the current I and, thus, for extension of the maximum time span the time-varying magnetic field H can be generated with the same amount of energy.

For stimulation of a tissue, in particular for defibrillation, a minimum time span of more than 2,5 ms, preferably more than 3 ms, in particular more than 4 ms, is preferred. Thus, coil 15 preferably is adapted such that a current I or current variation is limited to such extent that this minimum duration of the time-varying magnetic field H is achieved.

The stimulation device 1 can comprise a control device 16 which is adapted to control the time-varying magnetic field H, in particular by starting generation of plasma 3, by stopping current I flowing through plasma 3 and/or by influencing the strength of current I. For instance, control device 16 can apply a high voltage to the ionization electrode 10 for generating the plasma 3 inside the vessel 7. The current through plasma 3 can be limited by control device 16 in order to control the time-varying magnetic field or can be stopped to terminate the plasma 3.

The control device 16 can comprise an energy storing device 17 or an energy storing device 17 can be assigned to control device 16. It is preferred that the energy storing device 17 is adapted to store energy of more than 10 kJ, preferably more than 50 kJ, in particular more than 100 kJ. This amount of energy can be sufficient for directly defibrillating without a need of additional components like a remote device and/or electrode device 4. The energy storing device 17 and/or the electrode device 4 can be adapted to provide a capacity of more than 1 mF, preferably more than 2 mF, in particular more than 5 mF or 10 mF.

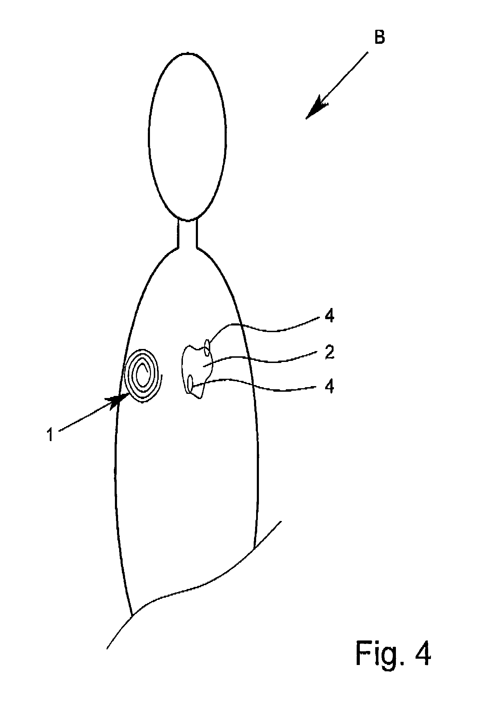

Fig. 4 shows a simplified example of a stimulation system according to the present invention implanted into a body B. The stimulation device 1 preferably is implanted, in particular under the skin of the thorax of a patient. In addition, two or more electrode devices 4 can be implanted, in particular in the vicinity of the heart.

Alternatively or additionally, the stimulation device 1 can be placed outside the body B, preferably close to the skin of the thorax. It is preferred to make use of at least one implanted electrode device 4. However, any electrode device 4 is optional and it is preferred, that at least one of pacing and defibrillation can be performed directly by the stimulation device 1 as described above. Alternatively, the stimulation device 1 does not need to be adapted for direct stimulation as long as at least one electrode device 4 is provided.

The magnetic field H preferably reaches, in particular affects, at least one, two or more of the electrode devices 4 such that the electrode devices 4 can generate an electrical impulse 5 for stimulation, in particular defibrillation, of a surrounding tissue, in particular a heart in the example shown. For a defibrillation it is preferred that at least 70 % of the tissue of a heart is depolarized. Therefore it is preferred that the electrode device 4 is adapted to store and/or the stimulation device 1 is adapted to provide via the time-varying magnetic field H energy, in particular to a sample, tissue or heart that is to be depolarized, of more than 1 J, preferably more than 3 J, in particular more than 5 J. If pacing is intended, this energy can be a factor of 10, 20 or 50 smaller. In the sense of the present invention, the term "pacing" preferably relates to electric triggering of heart beats.

The plasma 3 which is conducting current I can be used for generating the time-varying magnetic field H and for controlling and/or supplying with energy of the electrode device 4 for defibrillation and/or pacing in an exclusively wireless manner by means of this time-varying magnetic field H.

However, the stimulation device 1 and/or the stimulation system of the present invention preferably is or are not limited to stimulating a tissue but can be used for different stimulation or other purposes as well. For instance, the stimulation device 1 or the stimulation system can be used for stimulating samples in nuclear spin analysis or for stimulating electrochemical processes such as for storing energy or for analysis purposes as well. Thus, "stimulation" according to the present invention preferably is understood in a broad sense and is not limited to manipulating cells 6. In particular, any use or process described herein can be applied to different fields of technology and is or are not limited to stimulation of a tissue 2, body B, cells 6 or the like.

According to a further embodiment of the present invention, the stimulation device 1 and/or the stimulation system can be used in different fields of technology and even if stimulation is not the primary purpose. For example, devices for electrolysis or electroanalysis can be supplied with energy and/or can be controlled in a wireless manner with the stimulation device 1 according to the present invention. In a further example, a transportation system using a linear drive can be supplied with energy and/or can be controlled in a wireless manner by the stimulation device 1 according to the present invention. In this example, a solenoid drive can be stimulated by the stimulation device 1 or electrochemical processes can be stimulated by stimulation device 1 for storing energy or the like. In particular, even without any stimulation, stimulation device 1 can be used for supplying and/or controlling.

The time-varying magnetic field H generated by the plasma 3 conducting current I can be used for controlling and/or supplying with energy of various remote devices, in particular of one or more electrode devices 4, in an exclusively wireless manner by means of this time-varying magnetic field H as well. This can lead to the same or similar features and advantages as described above, e.g. to a lightweight and cheap realization of the stimulation device 1 or a system comprising stimulation device 1. Aspects and advantages described above can apply as well, even if no tissue is stimulated. Further, different aspects of this invention can be realized separately or in any combination, the combinations covering stimulation of a tissue, a remote device and/or a sample simultaneously by the stimulation device 1.

The stimulation device 1 can comprise or can be formed as a handheld tool. Preferably, this handheld tool at least comprises the plasma 3 and/or the superconductor for generating the time-varying magnetic field H and can be connected to a stationary part of stimulation device 1.

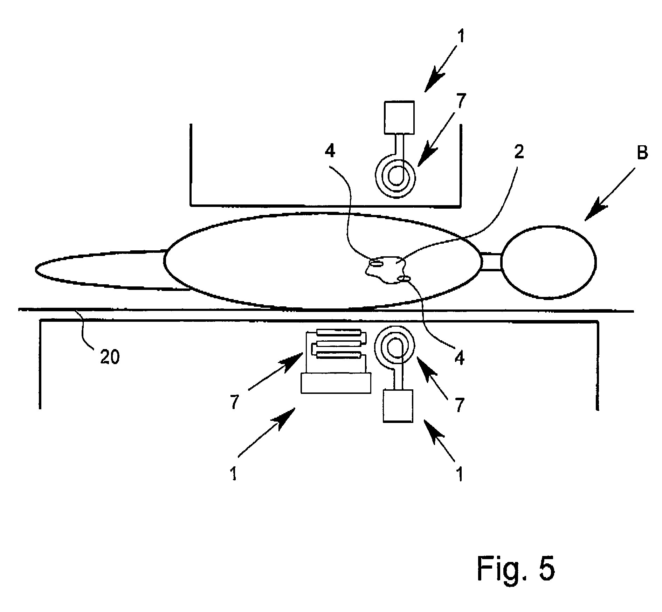

Fig. 5 shows a body B on a couch 20 as well as stationary and/or external stimulation device 1. The stationary stimulation device 1 shown in Fig. 5 preferably is adapted for directly stimulating, in particular defibrillating, tissue 2, in particular a heart, or a different sample. At least one, preferably two or more stimulation devices 1 can be provided, in particular synchronized and/or placed at different sides of a body B or different sample. Alternatively or additionally, the stimulation device 1 can comprise two or more separate plasmas 3, vessels 7 and/or superconductors, in particular each forming a separate coil. In the example shown, separate plasma vessels 7, each of at least one, preferably more than two windings, are used. Vessels 7 in the example shown can be wound and/or linear. Moreover, multiple vessels 7 can be connected forming a joint stimulation device 1.

Alternatively or additionally, one, two or more of the stimulation devices 1 and/or separate plasmas 3, vessels 7 and/or superconductors can be placed in handheld tools for more variable applicability. In particular, such a handheld tool can be connected to stationary equipment, in particular to stationary stimulation device 1 shown in Fig. 5. At least one of the stimulation devices 1 in Fig. 5 can be implemented as a handheld device.

The stimulation device 1 according to the present invention preferably allows for working through insulating materials like fabric, cloth or sample carriers. Thus, the present invention favorably allows for omitting undressing and/or application of electrodes to the skin of body B.

In the description of preferred embodiments, the present invention has been explained primarily by using plasma 3 for generating the time-varying magnetic field H. However, it preferably is to be understood that these or similar solutions can be provided using superconductors alternatively or additionally. In particular, vessel 7 can comprise, in particular cover, a superconductor material. Shielding layer 11 can be used for cooling the superconductor, in particular wherein shielding layer 11 can be a cooling means and/or can comprise or conduct a coolant liquid, in particular a liquid gas, and/or the layer 11 shields the superconductor from being warmed up. The ionization electrode 10 can act as an temperature insulator, preferably covering the superconductor. Superconductors can be used in addition to plasma 3 or vice versa. Further modifications might be provided if superconductors are used instead of or in addition to plasma 3.

**Reference numerals**
- 1: stimulation device
- 2: tissue
- 3: plasma
- 4: electrode device
- 5: electrical impulse
- 6: cells
- 7: vessel
- 8: wall
- 9: connecting electrode
- 10: ionization electrode
- 11: layer
- 12: diameter
- 13: cover
- 14: insulator
- 15: coil
- 16: control device
- 17: energy storing device
- 18: electrodes
- 19: receiving coil
- 20: couch

## Claims

1. Stimulation device (1) adapted to directly or indirectly stimulating a tissue (2) by means of a time-varying magnetic field (H), wherein the stimulation device (1) is adapted to generate the time-varying magnetic field (H) by means of a current (I) through a plasma (3) and/or a superconductor, and
wherein the stimulation device (1) is adapted for controlling and/or supplying with energy of an implanted electrode device (4) in an exclusively wireless manner by means of the time-varying magnetic field (H); and/or
wherein the stimulation device (1) is a defibrillator adapted for depolarization of cells (6) by means of the time-varying magnetic field (H).

2. Stimulation device of claim 1, **characterized in that** the stimulation device (1) comprises an elongate, preferably flexible, plasma vessel (7) comprising the plasma (3).

3. Stimulation device of claim 2, **characterized in that** the vessel (7) is filled with an ionizable gaseous medium, in particular an inert gas and/or a metal vapor.

4. Stimulation device of claim 2 or 3, **characterized in that** the vessel (7) comprises an electrically insulating wall (8) forming a gas tight interior of the vessel (7), in particular comprising the plasma (3).

5. Stimulation device of any one of the claims 2 to 4, **characterized in that** the vessel (7) comprises at least two connecting electrodes (9) at lengthwise opposing ends electrically connecting the plasma (3) and/or the interior to the exterior of the vessel (7).

6. Stimulation device of any one of the claims 3 to 5, **characterized in that** the vessel (7) comprises least one ionization electrode (10) that is arranged outside the vessel (7) electrically insulated from the interior of the vessel (7) and adapted to ionize the gaseous medium inside the vessel (7), in particular by means of an electrical field.

7. Stimulation device of any one of the claims 2 to 6, **characterized in that** the vessel (7) is adapted to block or reduce emission of electromagnetic radiation, preferably wherein the vessel (7) comprises a layer (11) of a material that conducts electrically and/or that blocks, converts, shields and/or reflects electromagnetic radiation, in particular wherein the layer (11) is an exterior coating of the vessel (7) and/or covers the interior of the vessel (7) at least substantially.

8. Stimulation device of any one of the claims 2 to 7, **characterized in that** the layer (11) blocks, converts, shields and/or reflects light, UV, X-rays, Gamma rays, and/or radiations with frequencies higher than 10⁹ Hz, preferably higher than 10" Hz, in particular higher 10¹³ Hz and/or lower than 10¹⁸ Hz, preferably lower than 10¹⁷ Hz, in particular lower than 10¹⁶ Hz.

9. Stimulation device of any one of the claims 2 to 8, **characterized in that** the vessel (7) is wound, preferably comprising at least two turns, in particular wherein the turns of the vessel (7) have a diameter of more than 2 cm, preferably more than 3 cm, in particular more than 5 cm, and/or less than 30 cm, preferably less than 20 cm, in particular less than 15 cm.

10. Stimulation device of any one of the claims 2 to 9, **characterized in that** the vessel (7) in its cross-section has an inside diameter (12) of less than 5 mm, preferably less than 2 mm, in particular less than 1 mm and/or an outer diameter of less than 7 mm, preferably less than 5 mm, in particular less than 3 mm.

11. Stimulation device of any one of the claims 2 to 10, **characterized in that** stimulation device (1) is implantable.

12. Stimulation device of any one of the claims 2 to 11, **characterized in that** the plasma (3) is electrically connected via a coil (15), in particular a choke, wherein the stimulation device (1) is adapted such that the current (I) through the plasma (3) has to pass the coil (15) as well.

13. Stimulation device of any one of the claims 2 to 12, **characterized in that** the stimulation device (1) comprise a control device (16) adapted to control the time-varying magnetic field (H), in particular by starting generation of the plasma (3), by stopping the current (1) through the plasma (3) and/or by influencing the strength of the current (I).

14. Stimulation system comprising a stimulation device (1) according to any one of the preceding claims, wherein the stimulation device (1) is adapted to generate a time-varying magnetic field (H) by means of a current (I) flowing through a plasma (3) and/or a superconductor, wherein the stimulation system further comprises an implantable electrode device (4), the electrode device (4) comprising electrodes (18) for delivery of an electrical impulse (5), preferably to a surrounding tissue (2), wherein the electrode device (4) is adapted to be controlled and/or supplied with energy exclusively in a wireless manner by means of the time-varying magnetic field (H).

15. Use of plasma (3) and/or of a superconductor conducting a current (I) for generating a time-varying magnetic field (H) and for controlling and/or supplying with energy of a remote device, in particular an electrode device (4), in an exclusively wireless manner by means of this time-varying magnetic field (H).
